# EUROPÄISCHE PATENTSCHRIFT

(11) **EP 0 861 229 B1**
(45) Veröffentlichungstag und Bekanntmachung des Hinweises auf die Patenterteilung: **23.01.2002**
(21) Anmeldenummer: 96937219.2
(22) Anmeldetag: 24.10.1996
(51) Int. Cl.: C07C 251/60, C07C 271/28, A01N 37/50, A01N 37/36, A01N 47/20, C07C 251/40, C07C 251/48

(54) **PHENYLESSIGSÄUREDERIVATE, VERFAHREN UND ZWISCHENPRODUKTE ZU IHRER HERSTELLUNG UND IHRE VERWENDUNG ZUR BEKÄMPFUNG VON SCHADPILZEN UND TIERISCHEN SCHÄDLINGEN**
PHENYLACETIC ACID DERIVATIVES, PROCESS AND INTERMEDIATE PRODUCTS FOR THEIR PREPARATION, AND THEIR USE AS FUNGICIDES AND PESTICIDES
DERIVES D'ACIDE PHENYLACETIQUE, PROCEDES ET PRODUITS INTERMEDIAIRES PERMETTANT DE LES PREPARER, ET LEUR UTILISATION POUR LUTTER CONTRE LES PARASITES ANIMAUX ET LES CHAMPIGNONS NUISIBLES

(30) Priorität: 30.10.1995 DE 19540361
(43) Veröffentlichungstag der Anmeldung: 02.09.1998
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); OBERDORF, Klaus, D-69117 Heidelberg (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); MÜLLER, Bernd, D-67227 Frankenthal (DE); MÜLLER, Ruth, D-67159 Friedelsheim (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE); GÖTZ, Norbert, D-67547 Worms (DE); HARREUS, Albrecht, D-67063 Ludwigshafen (DE)
(86) Internationale Anmeldenummer: EP9604621
(87) Internationale Veröffentlichungsnummer: WO9716412

(56) Entgegenhaltungen:
- EP-A- 0 122 627
- EP-A- 0 201 221
- EP-A- 0 331 061
- WO-A-95/18789
- WO-A-95/21153
- WO-A-95/34526
- WO-A-96/32373
- DE-A- 2 553 595
- DE-A- 3 425 118
- BULLETIN OF THE CHEMICAL SOCIETY OF JAPAN, Bd. 35, Nr. 9, 1962, TOKYO JP, Seiten 1465-71, XP002022993 SHUNSUKE MURAHASHI ET AL.: "An attempt to prepare linear chelate polymers"
- JOURNAL OF ORGANIC CHEMISTRY, Bd. 46, Nr. 1, 1981, EASTON US, Seiten 134-40, XP002022994 J. M. DOMAGALA ET AL.: "Synthesis of (Z)-4-(acylamino)- and 4-(alkylamino)-.alpha.-oximinophenylacetic acids: properties and stereochemical determination"
- JOURNAL OF ORGANIC CHEMISTRY, Bd. 55, Nr. 6, 1990, EASTON US, Seiten 1669-72, XP002022995 J. L. C. MARAIS ET AL.: "Sodium Dichloroisocyanurate oxidation of a sterically hindered tetrahydro-9(10H)-acridinone"

## Beschreibung

Die vorliegende Erfindung betrifft Phenylessigsäurederivate der Formel I 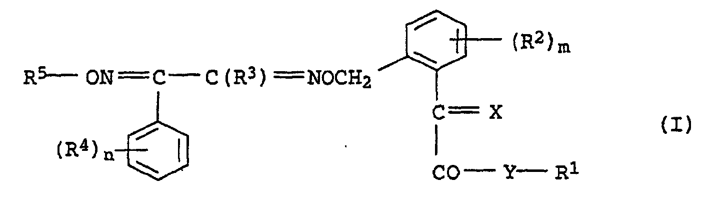 in der die Variablen die folgenden Bedeutungen haben:
- x: NOCH₃, CHOCH₃ oder CHCH₃;
- Y: O oder NH;
- R¹: Methyl;
- R²: Cyano, Nitro, Trifluormethyl, Halogen, C₁-C₄-Alkyl oder C₁-C₄-Alkoxy;
- m: 0, 1 oder 2, wobei die Reste R² verschieden sein können, wenn m für 2 steht;
- R³: Wasserstoff, Cyano, C₁-C₄-Alkyl, C₁-C₄-Halogenalkyl, C₁-C₄-Alkoxy oder Cyclopropyl;
- R⁴: C₁-C₄-Alkylendioxy, wobei die Alkylengruppen partiell oder vollständig halogeniert sind, oder einer der Reste:

-NR^{c}-(C=O)-Aₚ-R^{a},

wobei
R^{a} C₁-C₆-Alkyl oder C₂-C₆-Alkenyl;
R^{c} für Wasserstoff oder Alkyl, Alkenyl, Alkinyl, Alkylcarbonyl, Alkenylcarbonyl, Alkinylcarbonyl, Alkoxycarbonyl, Alkenyloxycarbonyl, Alkinyloxycarbonyl, Cycloalkyl, Cycloalkylcarbonyl, Cycloalkyloxycarbonyl, Arylcarbonyl oder Heteroarylcarbonyl steht;
p 0 oder 1 bedeutet und
A Sauerstoff, Schwefel oder Stickstoff bedeutet, wobei der Stickstoff Wasserstoff oder C₁-C₆-Alkyl trägt;
- n: 1 oder 2, wobei die Reste R⁴ verschieden sein können, wenn n für 2 steht;
- R⁵: Wasserstoff,
C₁-C₆-Alkylsulfonyl,
C₁-C₆-Alkyl, C₃-C₆-Alkenyl, C₃-C₆-Alkinyl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy, Heteroarylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamine, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy;
C₃-C₆-Cycloalkyl, welches partiell oder vollständig halogeniert sein und/oder unabhängig voneinander einen bis drei der folgenden Gruppen tragen kann: Cyano, C₁-C₆-Alkyl, C₁-C₆-Halogenalkyl, C₂-C₆-Alkenyl, C₁-C₆-Alkoxy und C₁-C₆-Alkylthio,
sowie deren Salze.

-N(R^{c})-OR^{d}, wobei

R^{a},R^{b} unabhängig voneinander stehen für Wasserstoff oder jeweils gegebenenfalls substituiertes Alkyl, Alkenyl, Alkinyl, Cycloalkyl, Cycloalkenyl, Heterocyclyl, Aryl oder Heteroaryl,
R^{c},R^{d} unabhängig voneinander stehen für Wasserstoff oder jeweils gegebenenfalls substituiertes Alkyl,Alkenyl, Alkinyl, Alkylcarbonyl, Alkenylcarbonyl, Alkinylcarbonyl, Alkoxycarbonyl, Alkenyloxycarbonyl, Alkinyloxycarbonyl, Cycloalkyl, Cycloalkylcarbonyl, Cycloalkyloxycarbonyl, Arylcarbonyl oder Heteroarylcarbonyl,
p 0 oder 1 bedeutet und
A Sauerstoff, Schwefel oder Stickstoff bedeutet, wobei der Stickstoff Wasserstoff oder C₁-C₆-Alkyl trägt;
- n: 1 oder 2, wobei die Reste R⁴ verschieden sein können, wenn n für 2 steht;
- R⁵: Wasserstoff,
C₁-C₆-Alkylsulfonyl,
C₁-C₆-Alkyl, C₃-C₆-Alkenyl, C₃-C₆-Alkinyl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy, Heteroarylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogen-Außerdem betrifft die Erfindung Verfahren und Zwischenprodukte zur Herstellung dieser Verbindungen sowie deren Verwendung zur Bekämpfung tierischer Schädlinge und Schadpilze.

Aus der Literatur sind Phenylessigsäurederivate zur Schädlingsbekämpfung bekannt (vgl. WO-A 95/18789. WO-A 95/21153, WO-A 95/21154), welche jedoch hinsichtlich ihrer Wirkung noch nicht befriedigen können.

Der vorliegenden Erfindung lagen daher neue Verbindungen dieses Typs mit verbesserter Wirkung als Aufgabe zugrunde.

Demgemäß wurden die eingangs definierten Phenylessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich bekannten Verfahren erhältlich.

Grundsätzlich ist es bei der Synthese der Verbindungen I unerheblich, ob zunächst die Gruppierung -C(X)-CO-Y-R¹ oder die Gruppierung 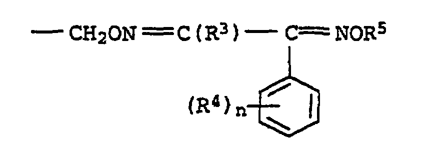 aufgebaut wird.

Der Aufbau der Gruppierung -C(X)-CO-Y-R¹ ist beispielsweise aus den eingangs zitierten, sowie aus folgenden Druckschriften bekannt: EP-A 242 070, EP-A 254 426, EP-A 370 629, EP-A 398 692, EP-A 422 597, EP-A 463 488, EP-A 472 300, EP-A 513 580, EP-A 656 352, DE-Anm. P 44 20 416.7.

Man geht beim Aufbau der Gruppierung 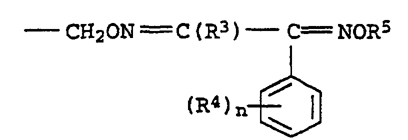 im allgemeinen so vor, daß man ein Benzylderivat der Formel II mit einem Hydroxyimin der Formel III umsetzt.

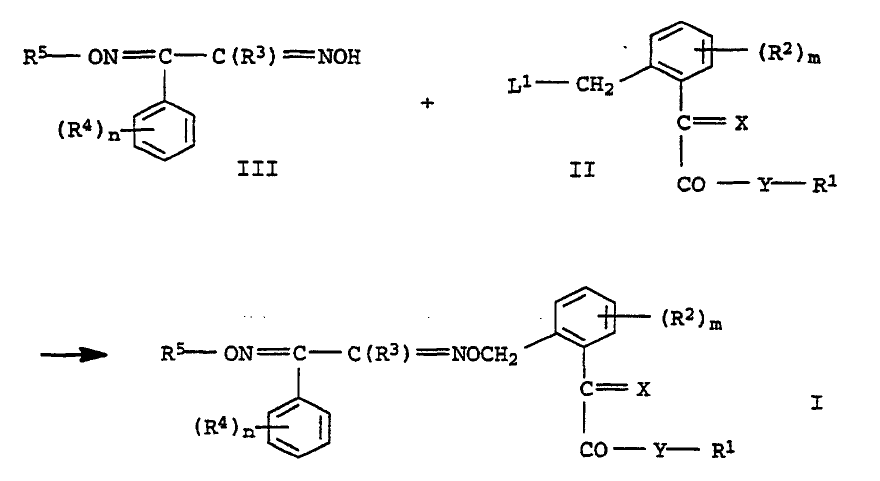

L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.

Die Umsetzung erfolgt in an sich bekannter Weise in einem inerten organischen Lösungsmittel in Gegenwart einer Base, z.B. Natriumhydrid, Kaliumhydroxid, Kaliumcarbonat oder Triethylamin gemäß den in Houben-Weyl, 4. Auflage, Bd. E 14b, S. 370 ff. und ibd. Bd. 10/1, S. 1189 ff. beschriebenen Methoden.

Das benötigte Hydroxyimin III erhält man beispielsweise durch Umsetzung eines entsprechenden Dihydroxyimins IV mit der Verbindung der Formel VI

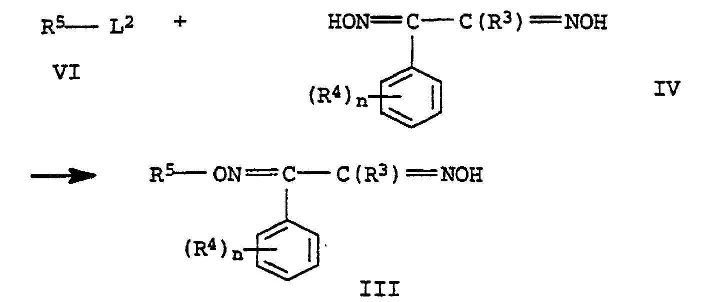

L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.

Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin oder Triethylamin gemäß: Houben-Weyl, 4. Auflage, Bd. E 14b, S. 307 ff., S. 370 ff. und S. 385 ff.; ibid., 4. Auflage, Bd. 10/4, S. 55 ff., S. 180 ff. und S. 217 ff.; ibid., 4. Auflage, Bd. E 5, S. 780 ff.

Die Verbindungen der Formel IV sind bekannt oder können nach an sich bekannten Methoden hergestellt werden (vgl. Gazz. Chim. Ital. 59, 719 (1929); Collect. Bull. Soc. Chim. Fr. 17, 71 (1897)).

Alternativ können die Verbindungen I auch dadurch erhalten werden, daß das Benzylderivat II zunächst mit dem Dihydroxyiminoderivat IV in ein entsprechendes Benzyloxim der Formel V umgesetzt wird, wobei V anschließend mit einer Verbindung einer Formel VI zu I umgesetzt wird.

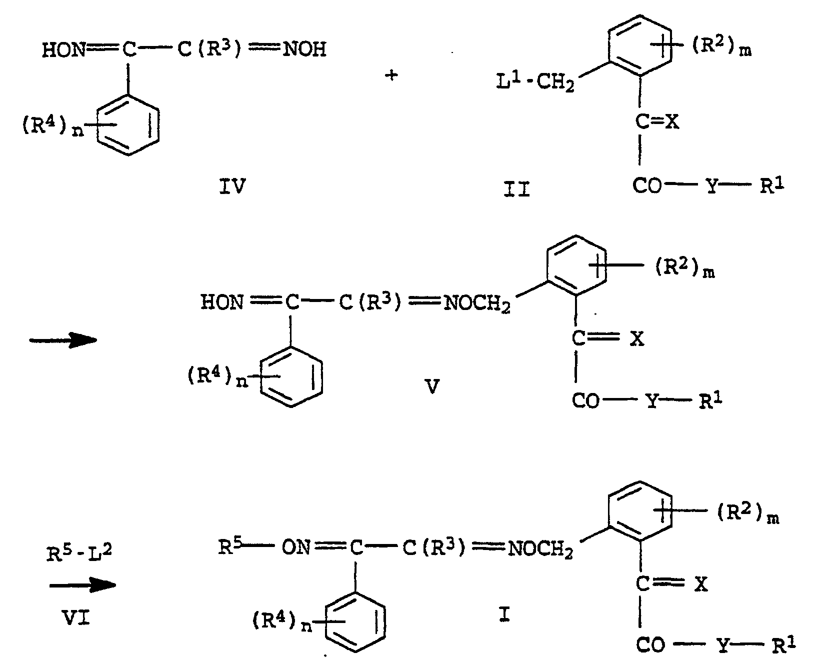

Die Umsetzung erfolgt in an sich bekannter Weise in einem inerten organischen Lösungsmittel in Gegenwart einer Base z.B. Kaliumcarbonat, Kaliumhydroxid, Natriumhydrid, Pyridin oder Triethylamin gemäß den in Houben-Weyl, 4. Auflage, Bd. 10/1, S. 1189 ff., Bd. E 14b, S. 307 ff., S. 370 ff. und S. 385 ff., Bd. 10/4, S. 55 ff., S. 180 ff. und S. 217 ff., Bd. E 5, S. 780 ff., beschriebenen Methoden.

Analog ist es ebenfalls möglich, das benötigte Hdroxyimin der Formel III aus einem Carbonylhydroxyimin VII durch Umsetzung mit einem Hydroxylamin IXa oder seinem Salz IXb herzustellen.

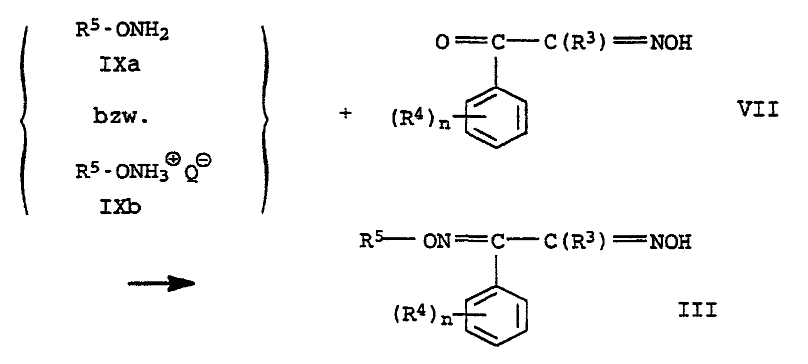

Q^{⊖} in der Formel IXb steht für das Anion einer Säure, insbesondere einer anorganischen Säure, z.B. Halogenid wie Chlorid.

Die Umsetzung erfolgt in an sich bekannter Weise in einem inerten organischen Lösungsmittel gemäß den in EP-A 513 580; Houben-Weyl, 4. Auflage, Bd. 10/4, S. 73 ff., Bd. E 14b, S. 369 ff. und S. 385 ff. beschriebenen Methoden.

Die Verbindungen der Formel VII sind bekannt oder können nach an sich bekannten Methoden hergestellt werden (J. Am. Pharm. Assoc. 35, 15 (1946)).

Alternativ können die Verbindungen I auch dadurch erhalten werden, daß das Benzylderivat II zunächst mit dem Carbonylhydroxyiminoderivat VII in ein entsprechendes Benzyloxyimin der Formel VIII umgesetzt wird, wobei VIII anschließend mit dem Hydroxylamin IXa bzw. dessen Salz IXb zu I umgesetzt wird.

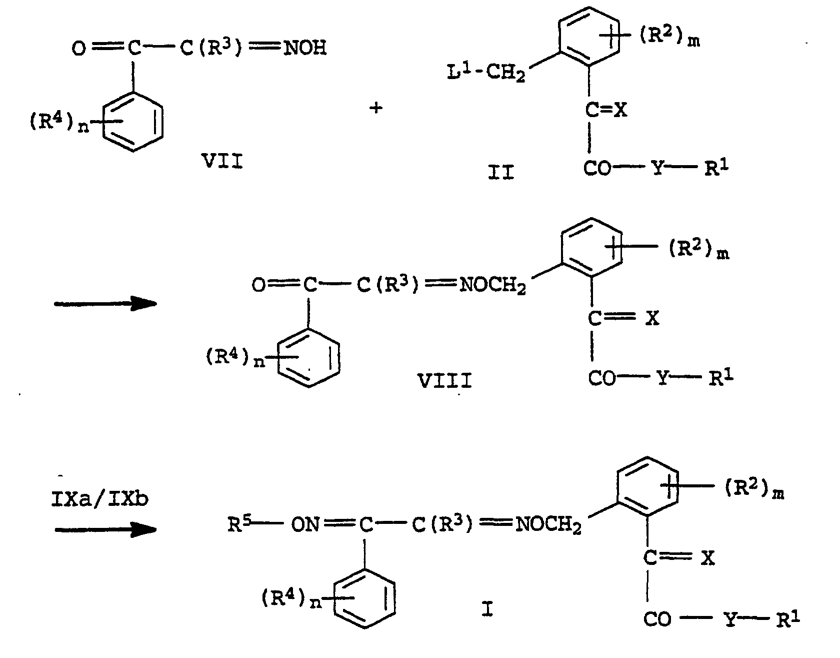

Die Umsetzung erfolgt in an sich bekannter Weise in einem inerten organischen Lösungsmittel gemäß den in Houben-Weyl, 4. Auflage, Bd. E 14b, S. 369 ff., Bd. 10/1, S. 1189 ff. und Bd. 10/4, S. 73 ff. oder EP-A 513 580 beschriebenen Methoden.

Eine weitere Möglichkeit zur Herstellung der Verbindungen I, bei denen R³ nicht C₁-C₄-Alkoxy bedeutet, ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung X.

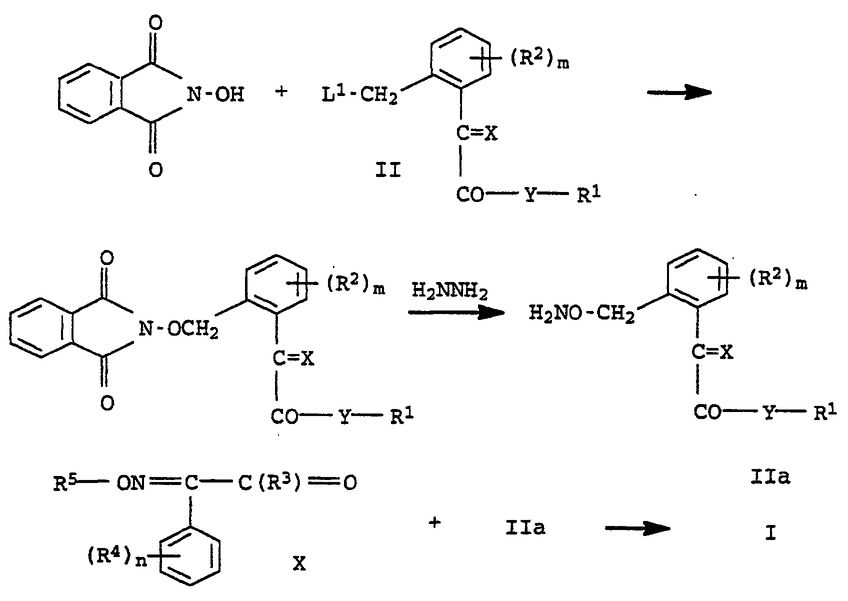

Die Umsetzung erfolgt in an sich bekannter Weise in einem inerten organischen Lösungsmittel gemäß den in EP-A 463 488 und EP-A 585 751 beschriebenen Methoden.

Die benötigte Carbonylverbindung X erhält man beispielsweise durch Umsetzung einer entsprechenden Hydroxyiminocarbonylverbindung VIIa mit einer Verbindung der Formel VI 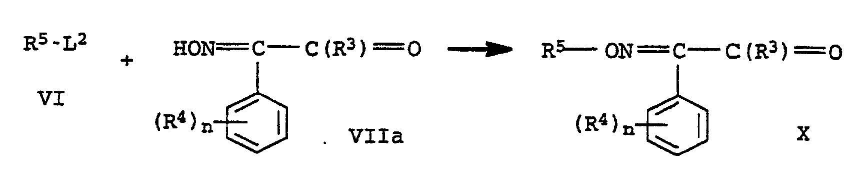 oder durch Umsetzung einer entsprechenden Dicarbonylverbindung XI mit einem Hydroxylamin IXa oder dessen Salz IXb

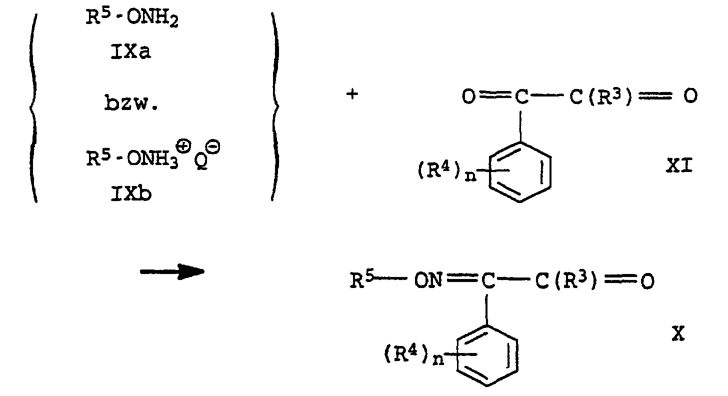

Die Umsetzungen erfolgen in an sich bekannter Weise in einem inerten organischen Lösungsmittel gemäß den in EP-A 513 580, Houben-Weyl, 4. Auflage, Bd. 10/4, S. 55 ff., S. 73 ff., S. 180 ff. und S. 217 ff., Bd. E 14b, S. 307 ff und 369 ff, Bd. E 5, S. 780 ff. beschriebenen Methoden.

Die Verbindungen der Formel VIIa bzw. XI sind bekannt oder können nach an sich bekannten Methoden hergestellt werden (J. Chem. Soc., 3094 (1955); Bull. Soc. Chim. Fr., 2894 (1969); Tetrahedron 40, 2035 (1984).

Entsprechend können die Verbindungen I auch dadurch erhalten werden, daß das Benzylhydroxylamin IIa zunächst mit dem Hydroxyiminoderivat VIIa in das entsprechende Benzyloxyiminoderivat der Formel V umgesetzt wird, wobei V anschließend mit einer Verbindung der Formel VI, wie vorstehend beschrieben, zu I umgesetzt wird.

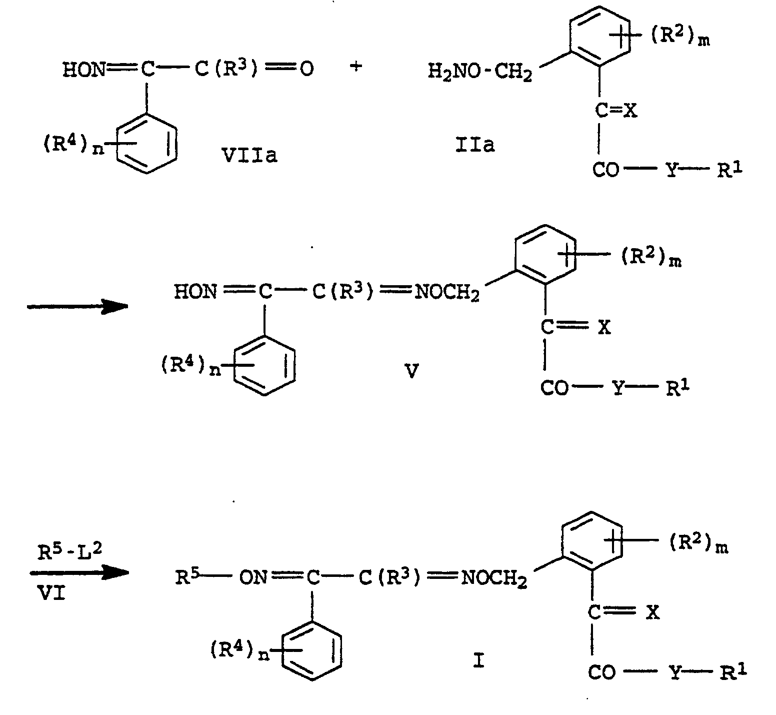

Analog können die Verbindungen I ebenfalls dadurch hergestellt werden, daß das Benzylhydroxylamin IIa zunächst mit dem Dicarbonylderivat der Formel XI in das Benzyloxyiminoderivat der Formel VIII überführt wird und VIII anschließend mit dem Hydroxylamin IXa oder dessen Salz IXb wie vorstehend beschrieben zu I umgesetzt wird.

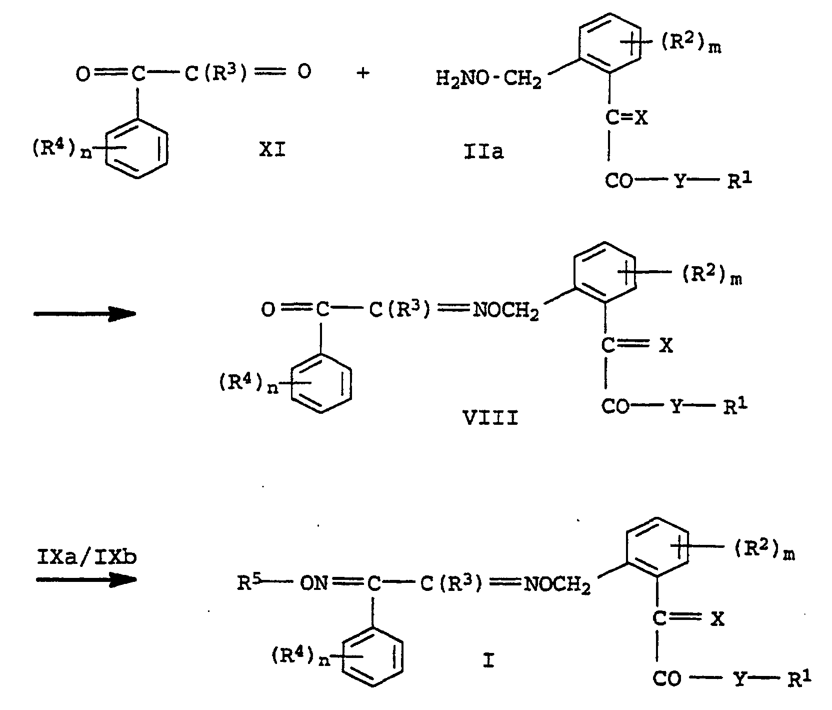

Des weiteren erhält man die Verbindungen I auch dadurch, daß man eine Verbindung III gemäß den in EP-A 493 711 beschriebenen Methoden mit einem Lacton XII zunächst in die entsprechende Benzoesäure XIII überführt und XIII über die entsprechenden Halogenide in die Cyanocarbonsäuren XIV überführt, welche im Wege der Pinner-Reaktion (Angew. Chem. 94, 1 (1982)) in die α-Ketoester XV überführt und ggf. weiter zu den α-Ketoamiden XVI umgesetzt werden (vgl. EP-A 348 766, DE-A 37 05 389, EP-A 178 826, DE-A 36 23 921, Houben-Weyl, 4. Auflage, Bd. E5, S. 941 ff.). 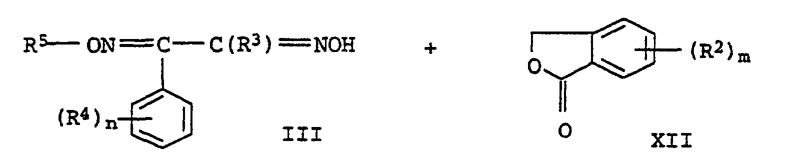 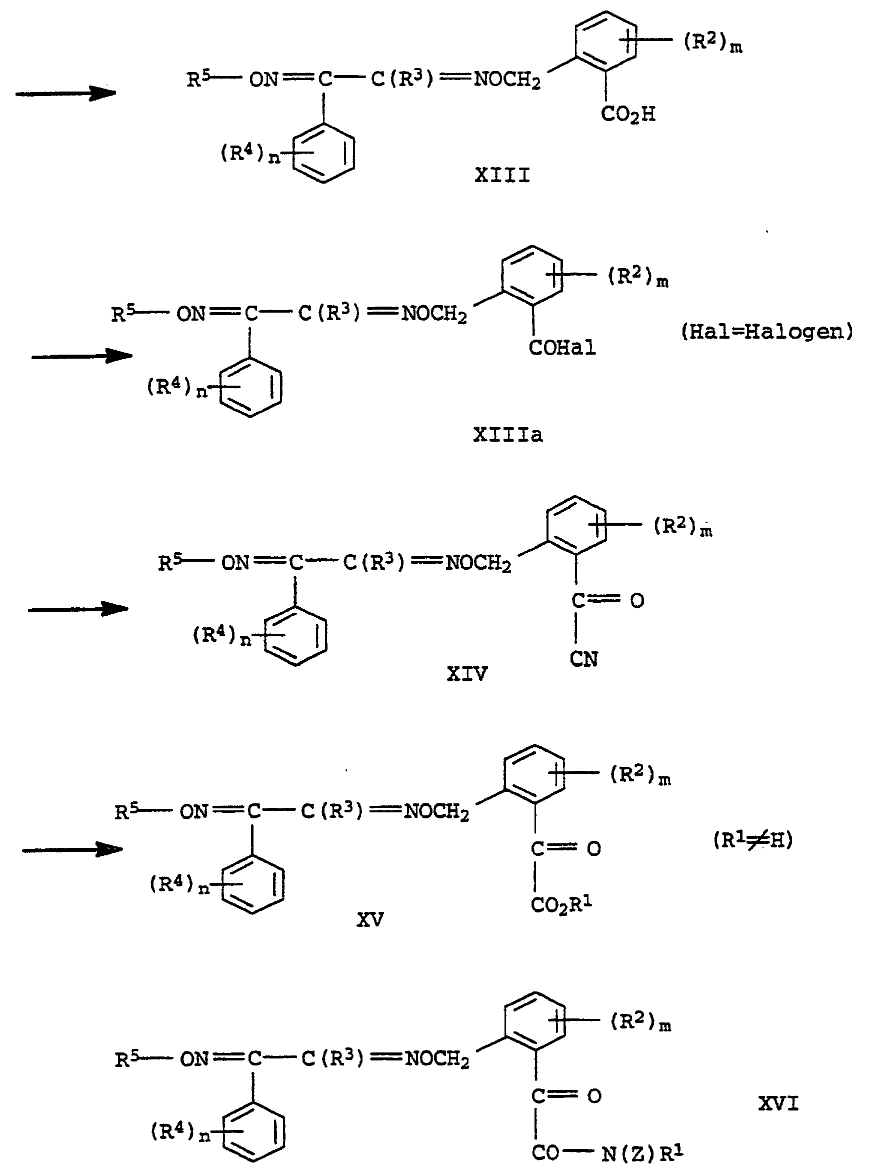 (Z = H oder C₁-C₄-Alkyl)

Die α-Ketoester XV und die α-Ketoamide XVI können gemäß üblicher Verfahren in die Verbindungen I überführt werden (vgl. EP-A 178 826, EP-A 513 580, DE-A 36 23 921, EP-A 398 692).

Verbindungen I, in denen R¹ Wasserstoff bedeutet,⁻erhält man nach diesem Verfahren durch Verseifung der Ester XV und anschließende Umsetzung zu I.

Die Verbindungen I, bei denen R⁴ für -C(=NOR^{a})-R^{b}, -NR^{c}-(C=O)-Aₚ-R^{a}, -O-(C=O)-NR^{a}R^{b} oder -N(R^{c})-OR^{d} steht, werden vorzugsweise ausgehend von den Verbindungen XVII, XVIII, XIX oder XX nach allgemein bekannten Methoden dargestellt.

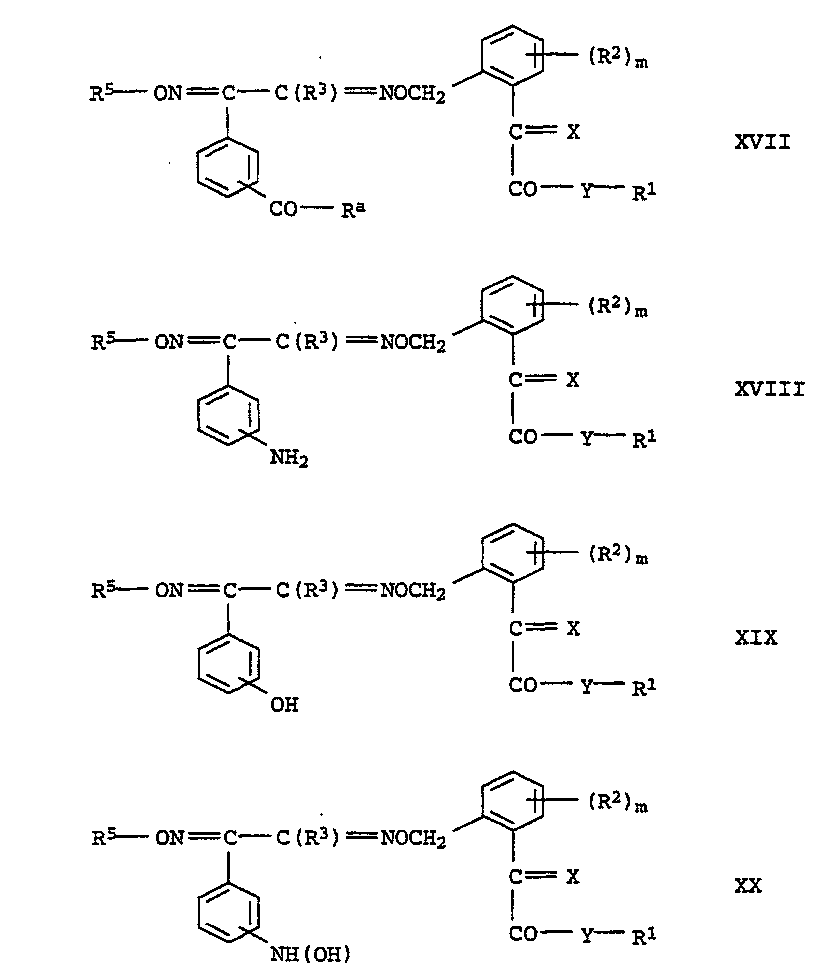

Die Verbindungen I, in denen Y für NH steht, können auch aus den entsprechenden Estern (Y=O) durch Umsetzung mit Aminen der Formel R¹NH₂ erhalten werden.

Die Verbindungen II sind bekannt (EP-A 513 580, BP-A 477 631, EP-A 463 488, EP-A 251 082, EP-A 400 417, EP-A 585 751) oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In bezug auf die C=X Doppelbindung werden hinsichtlich ihrer Wirksamkeit die E-Isomere der Verbindungen I bevorzugt (Konfiguration bezogen auf die -OCH₃ bzw. die -CH₃-Gruppe im Verhältnis zur -CO-Y-R¹-Gruppe).

In bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂- Gruppe).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen-mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
**Alkylendioxy:** divalente verzweigte oder unverzweigte Ketten aus 1-4 CH₂-Gruppen, die ggf. teilweise oder vollständig halogeniert sein können und wobei beide Valenzen über ein Sauerstoffatom an das Gerüst gebunden sind, z.B. OCH₂-O, O-CH₂CH₂-O, O-CHCl-CHCl-O-, O-(CH₂)₃-O;
**Cycloalkyl:** monocyclische Alkylgruppen mit 3 bis-6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoff atom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und. einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifacbbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Cycloalkyloxy bzw. Cycloalkylthio und Cycloalkylamino:** monocyclische Alkylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Cycloalkylcarbonyl:** Cycloalkylgruppen, wie vorstehend definiert, welche über eine Carbonylgruppe (-CO-) an das Gerüst gebunden sind;
**Cycloalkyloxycarbonyl:** Cycloalkyloxygruppen, wie vorstehend definiert, welche über eine Carbonylgruppe (-CO-) an das Gerüst gebunden sind;
**Alkenyloxycarbonyl:** Alkenyloxygruppen, wie vorstehend definiert, welche über eine Carbonylgruppe (-CO-) an das Gerüst gebunden sind;
**Alkinyloxycarbonyl:** Alkinyloxygruppen, wie vorstehend definiert, welche über eine Carbonylgruppe (-CO-) an das Gerüst gebunden sind;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Arylamino:** aromatische mono- oder polycyclische Kohlenwasserstoffreste, welche über ein Stickstoffatom an das Gerüst gebunden sind.
**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
   - 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
   bzw. die entsprechenden Oxy-, Thio-, Carbonyl- oder Sulfonylgruppen.
**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Aufgrund ihrer biologischen Wirksamkeit sind Verbindungen I bevorzugt, in denen R^{a} bedeutet:
C₁-C₆-Alkyl oder C₂-C₆-Alkenyl;
Ferner sind Verbindungen I bevorzugt, in denen R^{c} bedeutet:
Wasserstoff;
C₁-C₆-Alkyl, C₂-C₆-Alkenyl, C₂-C₆-Alkinyl, C₁-C₆-Alkylcarbonyl, C₂-C₆-Alkenylcarbonyl, C₂-C₆-Alkinylcarbonyl, C₁-C₆-Alkoxycarbonyl, C₂-C₆-Alkenyloxycarbonyl, C₂-C₆-Alkinyloxycarbonyl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy, Heteroarylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy;
C₃-C₆-Cycloalkyl, Cycloalkylcarbonyl, Cycloalkyloxycarbonyl, wobei diese Reste partiell oder vollständig halogeniert sein und/oder eine bis drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alxylthio;
Arylcarbonyl oder Heteroarylcarbonyl, wobei diese Reste ihrerseits partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxyl, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy und C₁-C₄-Alkylendioxy, welches halogeniert sein kann.

Außerdem sind Verbindungen I bevorzugt, in denen R⁵ bedeutet:
Wasserstoff;
C₁-C₆-Alkyl, C₂-C₆-Alkenyl, C₂-C₆-Alkinyl, wobei diese Reste partiell oder vollständig halogeniert sein oder einen bis drei der folgenden Gruppen tragen können: Cyano, Hydroxy, C₁-C₆-Alkoxy, C₁-C₆-Alkoxycarbonyl, C₁-C₆-Alkylthio, C₃-C₆-Cycloalkyl oder Oxiranyl, wobei die cyclischen Gruppen ihrerseits einen bis fünf der folgenden Substituenten tragen können: Halogen und C₁-C₄-Alkyl.
Hydroxy, C₁-C₆-Alkoxy, C₁-C₆-Halogenalkoxy, C₁-C₆-Alkoxycarbonyl, C₁-C₆-Alkylthio, C₂-C₆-Alkenyloxy, C₃-C₆-Cycloalkyl, C₃-C₆-Cycloalkyloxy, Heterocyclyl, Heterocyclyloxy, Aryl, Aryloxy, Heteroaryl, Heteroaryloxy, wobei die cyclischen Gruppen ihrerseits partiell oder vollständig halogeniert sein und/oder einen bis drei der folgenden Substituenten tragen können: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy und C₃-C₆-Alkinyloxy;
C₃-C₆-Cycloalkyl, Cycloalkylcarbonyl, Cycloalkyloxycarbonyl, wobei diese Reste partiell oder vollständig halogeniert sein und/oder eine bis drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alkylthio;
Arylcarbonyl oder Heteroarylcarbonyl, wobei diese Reste ihrerseits partiell oder vollständig halogeniert sein und/oder eine bis drei der folgenden Gruppen tragen können: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann.

Des weiteren sind Verbindungen der Formel I bevorzugt, in denen m für 0 steht.

Erfindungsgemäß sind Verbindungen der Formel I, in denen R¹ für Methyl steht.

Daneben werden Verbindungen I bevorzugt, in denen R³ für Wasserstoff, Cyclopropyl, Methyl, Ethyl, 1-Methylethyl, Methoxy, Cyano oder Trifluormethyl steht.

Besonders werden Verbindungen I bevorzugt, in denen R³ für Methyl steht.

Des weiteren werden Verbindungen I bevorzugt, in denen R³ für Methoxy steht.

Außerdem werden Verbindungen I bevorzugt, in denen R³ für Cyano steht.

Des weiteren werden Verbindungen I bevorzugt, in denen R³ für Trifluormethyl steht.

Des weiteren werden Verbindungen I bevorzugt, in denen n für 1 steht.

Außerdem werden Verbindungen I bevorzugt, in denen R⁴ für NR^{c}- (C=O) -Aₚ-R^{a} steht.

Erfindungsgemäß sind Verbindungen I bevorzugt, in denen R^{a} für C₁-C₆-Alkyl oder C₂-C₆-Alkenyl steht.

Des weiteren werden Verbindungen I bevorzugt, in denen R⁴ für C₁-C₄-Alkylendioxy steht, wobei die Alkylengruppen partiell oder vollständig halogeniert, bevorzugt fluoriert sind.

Besonders werden Verbindungen I bevorzugt, in denen R⁴ für Difluormethylendioxy steht.

Außerdem werden Verbindungen I bevorzugt, in denen R⁵ für Wasserstoff, C₃-C₆-Cycloalkyl, Arylalkyl, Hetarylalkyl, Aryloxyalkyl, Hetaryloxyalkyl steht.

Besonders werden Verbindungen I bevorzugt, in denen R⁵ für C₁-C₆-Alkyl steht.

Ganz besonders werden Verbindungen I bevorzugt, in denen R⁵ für Methyl oder Ethyl steht.

Des weiteren werden Verbindungen I bevorzugt, in denen R⁵ für C₁-C₆-Alkylsulfonyl steht.

Daneben werden Verbindungen der Formel I bevorzugt, in denen X für NOCH₃ steht.

Daneben werden Verbindungen der Formel I bevorzugt, in denen X für CHOCH₃ und Y für O steht.

Daneben werden Verbindungen der Formel I bevorzugt, in denen X für CHCH₃ und Y für O steht.

Außerdem werden Verbindungen der Formel I bevorzugt, in denen Y für O steht.

Desweiteren werden Verbindungen der Formel I bevorzugt, in denen Y für NH steht.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Gemüse und Zierpflanzen, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können mit an sich bekannten Formulierungshilfsmitteln in die üblichen Formulierungen (Mittel) überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der Verbindungen I gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl (2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-lH-1,2,4-triazol.

Strobilurine wie Methyl-E-methoximino-[α-(o-tolyloxy)-o-tolyl] -acetat, Methyl-E-2-{2-[6-(2-cyanophenoxy)-pyrimidin-4-yloxy]-phenyl}-3-methoxyacrylat, Methyl-E-methoximino-[α-(2-phenoxyphenyl)]-acetamid, Methyl-E-methoximino-[α-(2,5-dimethylphenoxy)-o-tolyl]-acetamid.

Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin.

Phenylpyrrole wie 4-(2,2-difluor-1,3-benzodioxol-4-yl)-pyrrol-3-carbonitril.

Zimtsäureamide wie 3-(4-Chlorphenyl)-3-(3,4-dimethoxyphenyl)-acrylsäuremorpholid.

(2RS,3RS)-1-[3-(2-Chlorphenyl)-2-[4-fluorphenyl]oxiran-2-yl-methyl]1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Verbindungen I können als solche, in Form ihrer unter Verwendung an sich bekannter Formulierungshilfsmittel gewonnener Formulierungen (Mittel) oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwekken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung I werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung I werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung I werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung I werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung I werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung I mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung I werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung I werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiel 1

### Synthese von Verbindung I.11 gemäß Tabelle 1

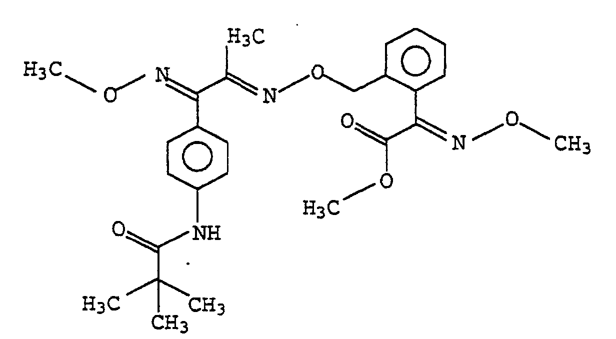

1a) Synthese von 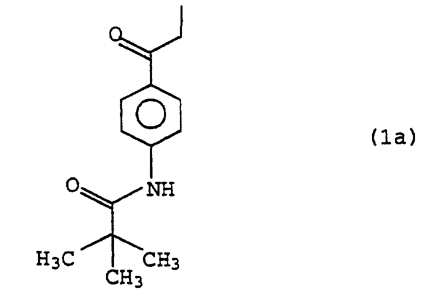
   Zu einer gerührten Mischung von 82 g (0,45 ml) Pivalinsäureanilid und 40 g (0,43 mol) Propionylchlorid wurden portionsweise 162 g (1,2 mol) AlCl₃ gegeben, wobei sich die Reaktionsmischung auf ca. 100°C erwärmte und Salzsäure ausgaste.
   Anschließend rührte man die Reaktionsmischung 6 Stunden bei 80°C. Die Reaktionsmsischung wurde vorsichtig auf Eis gegeben und die wäßrige Phase mit Methyl-t-butylether und Essigester extrahiert.
   Die vereinigten organischen Phasen wurden 2x mit Wasser extrahiert, über MgSO₄ getrocknet und eingeengt. Der Rückstand wurde säulenchromatographisch mit Cyclohexan/Essigester-Gemischen gereinigt. Man erhielt 22,3 g (22 %) der Titelverbindung la als farblosen Festkörper (Fp = 121o^{c)}.
   ¹H-NMR (CDCl₃; δ in ppm):
   7,95 (d, 2H, Phenyl); 7,65 (d, breit, 3H, NH, 2xPhenyl); 2,95 (q, 2H, CH₂); 1,35 (s, 9H, t-Butyl); 1,2 (t, 3H, CH₃)
1b) Synthese von 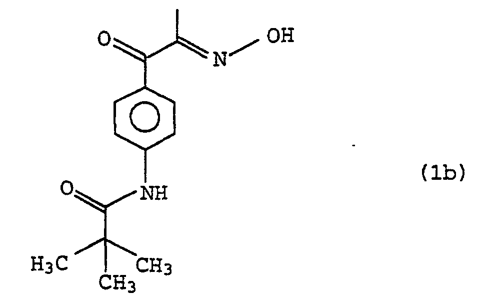
   Eine Mischung von 22 g (94 mmol) der Verbindung la in 200 ml Toluol wurde bei -20°C mit 80 ml etherischer Salzsäure-Lösung (ca. 4n) und anschließend tropfenweise mit 12 g (115 mmol) n-Butylnitrit in 50 ml Ether versetzt. Dann rührte man über Nacht bei Raumtemperatur. Die Titelverbindung kristallisierte aus der Reaktionsmischung aus. Der ausgefallene Festkörper wurde abgesaugt, mit Methyl-t-butylether, Essigester und Methylenchlorid gewaschen und mit Stickstoff trockengeblasen. Man erhielt 17 g (69 %) der Titelverbindung 1b als farblosen Festkörper (Fp=198°C). Die Aufarbeitung der Mutterlauge lieferte eine zweite Kristallfraktion von 3,5 g (14%).
   ¹H-NMR (DMSO-d₆; δ in ppm):
   12,3; 9,5 (2s, je 1H, OH, NH); 7,85 (d, 2H, Phenyl); 7,75 (d, 2H, PHenyl); 2,0 (s, 3H, CH₃); 1,25 (s, 9H, t-Butyl)
1c) Synthese von 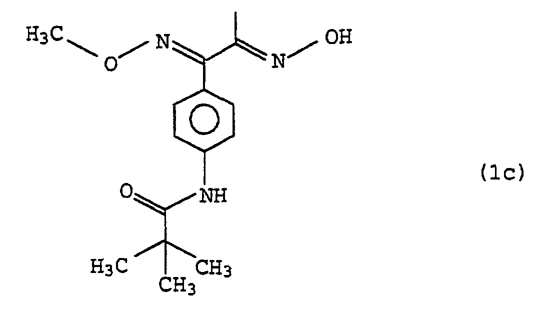
   Eine Mischung von 16 g (61 mmol) der Verbindung 1b, 12 g (150 mmol) Pyridin und 6,5 g (78 mmol) O-Methylhydroxylaminhydrochlorid in 100 ml Methanol wurde über Nacht bei Raumtemperatur gerührt. Anschließend wurde die Reaktionsmischung eingeengt und der Rückstand in Methylenchlorid aufgenommen. Die organische Phase wurde mit verd. Salzsäure und Wasser gewaschen, über MgSO₄ getrocknet und eingengt.
   Der Rückstand kristallisierte und wurde mit Methylenchlorid ausgerührt. Man erhielt 3,6 (20 %) der Titelverbindung 1c als farblosen Festkörper (Fp=208°C). Die Aufarbeitung der Mutterlauge lieferte eine zweite Kristallfraktion von 5,2 g 1c (29%).
   ¹H-NMR (DMSO-d₆; δ in ppm):
   11,6; 9,25 (2s, je 1H, OH, NH); 7,6 (d, 2H, Phenyl); 7,1 (d, 2H, Phenyl); 3,8 (s, 3H, OCH₃); 2,05 (s, 3H, CH₃); 1,25 (s, 9H, t-Butyl)

### Synthese von I.11

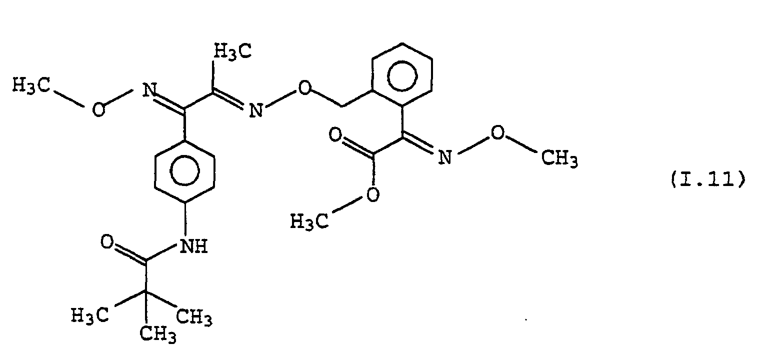

Eine Mischung von 2 g (6,8 mmol) der Verbindung 1c und 0,25 g (10 mmol) Natriumhydrid in 20 ml Dimethylformamid wurde 15 min bei Raumtemperatur gerührt. Anschließend wurden 2 g (7 mmol) 2-Brommethyl-phenylglyoxylsäuremethylester-trans-O-methyloxim (EP 254 426) hinzugegeben und eine Stunde bei Raumtemperatur gerührt. Sodann wurde die Reaktionsmischung mit Wasser verdünnt und die wäßrige Phase mit Methyl-t-butylether extrahiert. Die vereinigten organischen Phasen wurden mit Wasser gewaschen, über MgSO₄ getrocknet und eingeengt. Der Rückstand kristallisierte, wurde abgesaugt und mit Stickstoff trockengeblasen. Man erhielt 2,8 g (83 %) der Titelverbindung I11 als hellgelben Festkörper) (Fp=114-116°C).
¹H-NMR (CDCl₃-d₆; δ in ppm):
7,55; (2s, 2H, Phenyl); 7,4 (m, 4H, Phenyl); 7,15 (d, breit, NH, 2x Phenyl); 4,95 (s, 2H, OCH₂); 4,0; 3,9; 3,8 (3s, je 3H, 3xOCH₃); 2,1 (s, 3H, CH₃); 1,25 (s, 9H, t-Butyl)

### Synthesebeispiel 2

### Synthese von Verbindung I.12 gemäß Tabelle 1

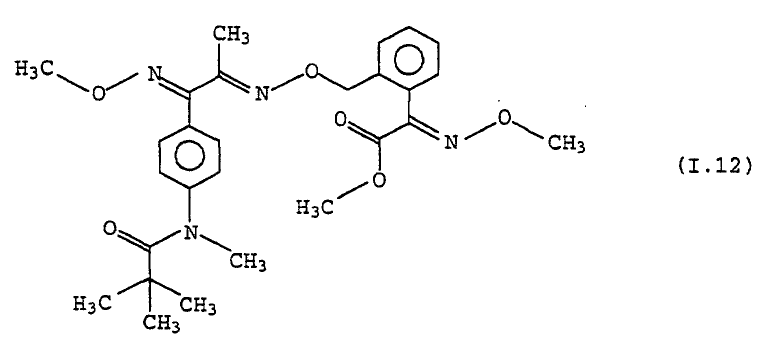

Eine Mischung von 1,5 g (3 mmol) der Verbindung I.11 gemäß Synthesebeispiel 1 und 0,1 g (4 mmol) Natriumhydrid in 20 ml Dimethylformamid wurde 10 min bei Raumtemperatur gerührt. Anschließend wurden 0,7 g (5 mmol) Methyljodid hinzugegeben und ca. 2 Stunden bei Raumtemperatur gerührt. Dann wurde die Reaktionsmischung mit Wasser verdünnt und die wäßrige Phase mit Methyl-t-butylether extrhiert. Die vereinigten organischen Phasen wurden mit Wasser extrahiert, über MgSO₄ getrocknet und eingeengt. Der Rückstand wurde säulenchromatographisch mit Cyclohexan/Essigester-Gemisch gereinigt. Man erhielt 1,2 g (78 %) der Titelverbindung I.12 als hellgelbe Kristalle (Fp = 111-113°C).
¹H-NMR (CDCl₃; δ in ppm):
7,35; (m, 2H, Phenyl); 7,2 (m, 6H, Phenyl); 4,9 (s, 2H, OCH₂); 4,0; 3,9; 3,8 (3s, je 3H, 3xOCH₃); 3,2 (s, 3H, NCH₃); 2,1 (s, 3H, CH₃); 1,05 (s, 9H, t-Butyl)

### Synthesebeispiel 3

### Synthese von Verbindung I.10 gemäß Tabelle 1

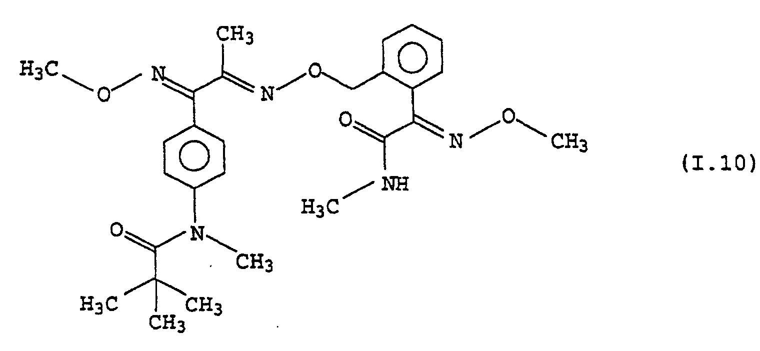

Eine Mischung von 0,9 g (1,8 mmol) der Verbindung I.12 gemäß Synthesebeispiel 2 und 3 ml Tetrahydrofuran in 20 ml 40%iger Methylamin-Lösung wurde 1 Stunde bei 50°C gerührt. Anschließend wurde das überschüssige Methylamin im Vakuum abgedampft und die zurückbleibende wäßrige Phase mit Methylenchlorid extrahiert. Die vereinigten organischen Phasen wurden mit Wasser gewaschen, über MgSO₄ getrocknet und eingeengt. Der Rückstand kristallisierte und wurde mit Methyl-t-butylether ausgerührt. Man erhielt 0,7 g (76 %) der Titelverbindung I.10 als farblosen Festkörper (Fp=168-170°C).
¹H-NMR (CDCl₃; δ in ppm):
7,35; (m, 2H, Phenyl); 7,2 {m, 6H, Phenyl); 6,75 (s, breit, 1H, NH); 4,9 (s, 2H, OCH₂); 3,9 (2s, je 3H, 2xOCH₃); 3,2 (s, 3H, NCH₃); 2,9 (d, 3H, HNCH₃); 2,1 (s, 3H, CH₃); 1,05 (s, 9H, t-Butyl)

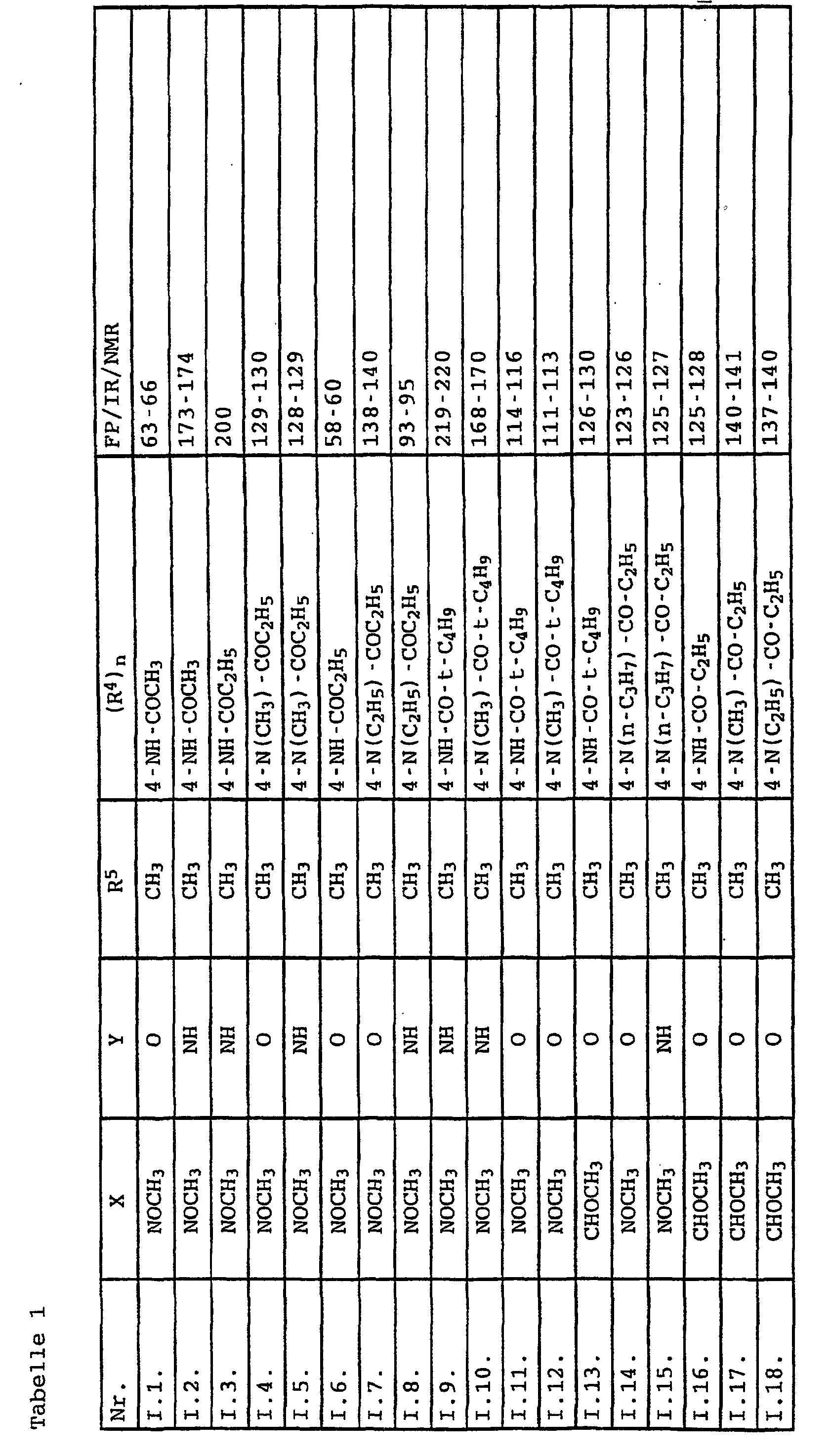

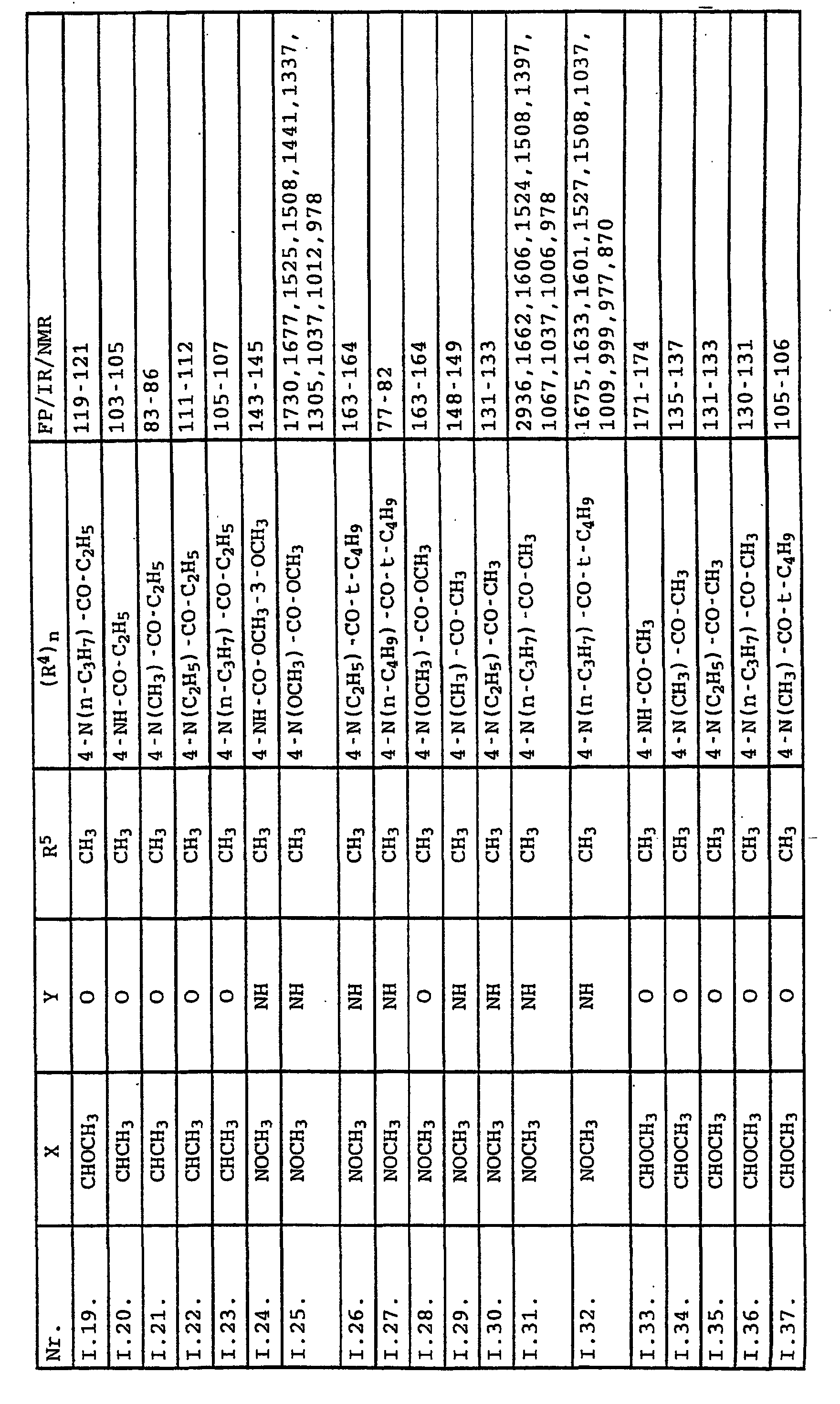

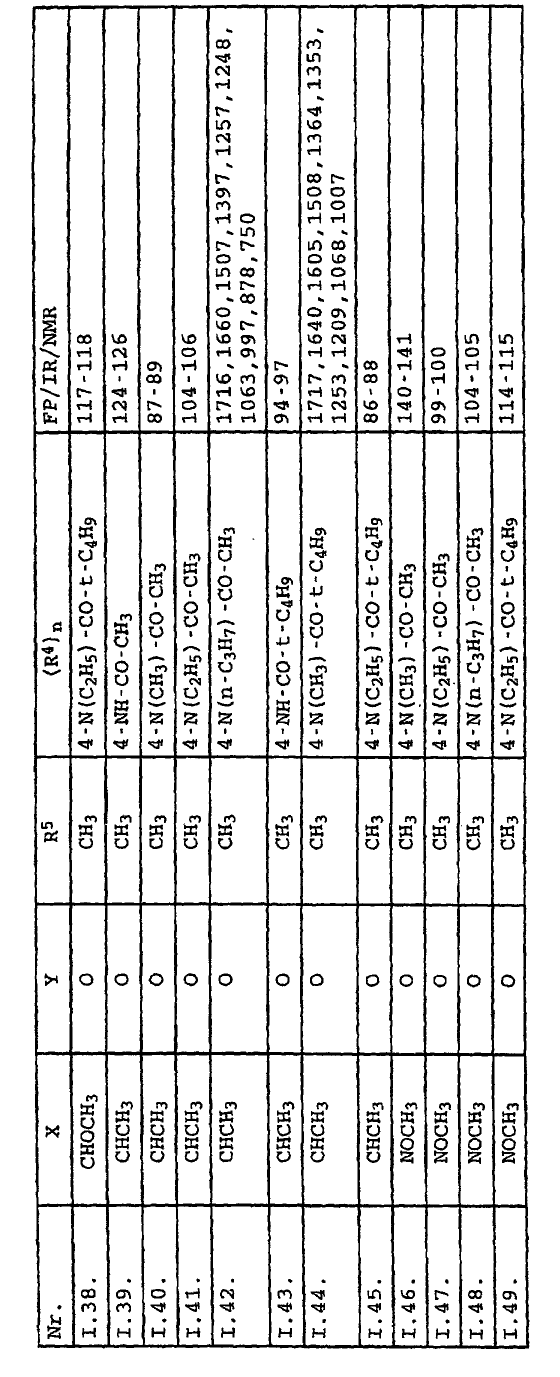

### Anwendungsbeispiele

Die fungizide Wirkung der Verbindungen der Formel I läßt sich durch folgende Versuche zeigen:

Die Wirkstoffe werden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt. Die Auswertung erfolgte visuell.

Die Wirkung der Verbindungen der allgemeinen Formel I gegen tierische Schädlinge läßt sich durch folgende Versuche zeigen

Die Wirkstoffe werden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

### Anwendungsbeispiel 1

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff gemäß Tabelle 1 und 20 % Emulgiermittel in der Trockensubstanz enthielt, besprüht und 24 Stunden nach dem Antrocknen des Spritzbelages mit Oidien (Sporen) des Weisenmehltaus (Erysiphe graminis var. tritici) bestäubt. Die Versuchspflanzen wurden anschließend im Gewächshaus bei Temperaturen zwischen 20 und 22°C und 75 bis 80% relativer Luftfeuchtigkeit aufgestellt. Nach 7 Tagen wurde das Ausmaß der Mehltauentwicklung ermittelt.

**Tabelle 2**

| Wirkstoff gemäß Tabelle 1 | %-Befall der Blätter nach Applikation von 250 ppm-haltiger wäßriger Wirkstoffaufbereitung |
|---|---|
| I.20 | 15 |
| I.21 | 5 |
| I.22 | 15 |
| I.23 | 15 |
| I.40 | 15 |
| I.41 | 15 |
| I.43 | 15 |
| I.45 | 15 |
| Vergleichssubstanz A (Nr. 1433 aus Tabelle 3 der WO 95/21153) | 30 |
| Nr. I.13 | 0 |
| Nr. I.16 | 5 |
| Nr. I.17 | 15 |
| Nr. I.18 | 5 |
| Nr. I.19 | 5 |
| Vergleichssubstanz B (Nr. 2 aus Tabelle II der WO 95/21153) | 30 |
| Vergleichssubstanz C (Nr.1433 aus Tabelle 1 der WO 95/21153) | 30 |
| Nr. I.12 | 5 |
| Vergleichssubstanz D (Nr. 48 aus Tabelle 1 der WO 95/21153) | 30 |
| Vergleichssubstanz E (Nr. 1901 aus Tabelle 7 der WO 95/21153) | 60 |
| Nr. I.5 | 5 |
| Nr. I.8 | 0 |
| Nr. I.15 | 5 |
| Nr. I.25 | 5 |
| Nr. I.26 | 15 |
| Nr. I.27 | 5 |
| Nr. I.31 | 15 |
| Nr. I.32 | 15 |
| Vergleichssubstanz F (Nr. 1898 aus Tabelle 5 der WO 95/21254) | 30 |
| Unbehandelt | 70 |

### Anwendungsbeispiel 2

### Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff gemäß Tabelle 1 und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

**Tabelle 3**

| Wirkstoff | %-Befall der Blätter nach Applikation von ... ppm-haltiger wäßriger Wirkstoffaufbereitung |
|---|---|
| | 63 ppm |
| Nr. I.40 | 0 |
| Nr. I.41 | 15 |
| Nr. I.43 | 5 |
| Nr. I.44 | 5 |
| Nr. I.45 | 15 |
| Vergleichssubstanz A (Nr. 1433 aus Tabelle 3 der WO 95/21153) | 40 |
| Nr. I.13 | 15 |
| Vergleichssubstanz B (Nr. 2 aus Tabelle II der WO 95/21153) | 60 |
| Vergleichssubstanz C (Nr.1433 aus Tabelle 1 der WO 95/21153) | 40 |
| | 63 ppm |
| Nr. I.12 | 15 |
| Vergleichssubstanz D (Nr. 48 aus Tabelle I der WO 95/21153) | 70 |
| Vergleichssubstanz E (Nr. 1901 aus Tabelle 7 der WO 95/21153) | 40 |
| Nr. I.5 | 0 |
| Nr. I.8 | 0 |
| | 16 ppm |
| Nr. I.13 | 5 |
| Nr. I.27 | 3 |
| Nr. I.29 | 5 |
| Nr. I.30 | 15 |
| Nr. I.31 | 0 |
| Nr. I.32 | 0 |
| Vergleichsprodukt F (Nr. 1898 aus Tabelle 5 der WO 95/21154). | 60 |
| Unbehandelt | 70 |

## Patentansprüche

1. Phenylessigsäurederivate der Formel I 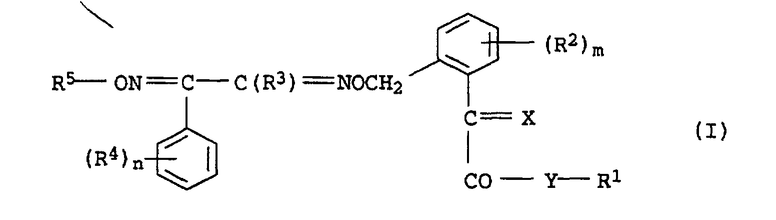 in der die Variablen die folgenden Bedeutungen haben:
X NOCH₃, CHOCH₃ oder CHCH₃;
Y O oder NH;
R¹ Methyl;
R² Cyano, Nitro, Trifluormethyl, Halogen, C₁-C₄-Alkyl oder C₁-C₄-Alkoxy;
m 0, 1 oder 2, wobei die Reste R² verschieden sein können, wenn m für 2 steht;
R³ Wasserstoff, Cyano, C₁-C₄-Alkyl, C₁-C₄-Halogenalkyl, C₁-C₄-Alkoxy oder Cyclopropyl;
R⁴ C₁-C₄-Alkylendioxy, wobei die Alkylengruppen partiell oder vollständig halogeniert sind, oder einer der Reste:
-NR^{c}-(C=O)-Aₚ-R^{a},
wobei
R^{a} C₁-C₆-Alkyl oder C₂-C₆-Alkenyl;
R^{c} für Wasserstoff oder Alkyl, Alkenyl, Alkinyl, Alkylcarbonyl, Alkenylcarbonyl, Alkinylcarbonyl, Alkoxycarbonyl, Alkenyloxycarbonyl, Alkinyloxycarbonyl, Cycloalkyl, Cycloalkylcarbonyl, Cycloalkyloxycarbonyl, Arylcarbonyl oder Heteroarylcarbonyl steht;
p 0 oder 1 bedeutet und
A Sauerstoff, Schwefel oder Stickstoff bedeutet, wobei der Stickstoff Wasserstoff oder C₁-C₆-Alkyl trägt;
n 1 oder 2, wobei die Reste R⁴ verschieden sein können, wenn n für 2 steht;
R⁵ Wasserstoff,
C₁-C₆-Alkylsulfonyl,
C₁-C₆-Alkyl, C₃-C₆-Alkenyl, C₃-C₆-Alkinyl, wobei diese Reste partiell oder vollständig halogeniert sein und/ oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Arylthio, Heteroaryl, Heteroaryloxy, Heteroarylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio und C₃-C₆-Alkinyloxy;
C₃-C₆-Cycloalkyl, welches partiell oder vollständig halogeniert sein und/oder unabhängig voneinander einen bis drei der folgenden Gruppen tragen kann:
Cyano, C₁-C₆-Alkyl, C₁-C₆-Halogenalkyl, C₂-C₆-Alkenyl, C₁-C₆-Alkoxy und C₁-C₆-Alkylthio,
sowie deren Salze.

2. Phenylsäurederivate der Formel I gemäß Anspruch 1, in der
R⁴ C₁-C₄-Alkylendioxy, wobei die Alkylengruppen partiell oder vollständig halogeniert sind, bedeutet.

3. Verfahren zur Herstellung der Verbindungen der Formel I gemäß Anspruch 1, **dadurch gekennzeichnet, daß** man ein Benzylderivat der Formel II 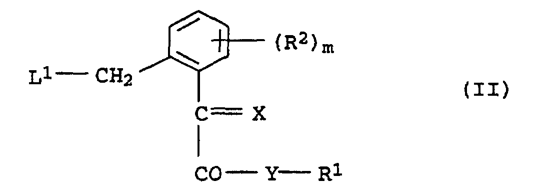 in der L¹ für eine nucleophil austauschbare Abgangsgruppe steht, mit einem Hydroxyimin der Formel III 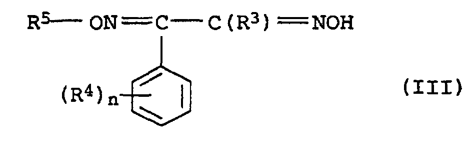 umsetzt.

4. Verfahren zur Herstellung der Verbindungen der Formel I gemäß Anspruch 1, **dadurch gekennzeichnet, daß** man ein Benzylderivat der Formel II gemäß Anspruch 1 in an sich bekannter Weise mit einem Dihydroxyimin der Formel IV 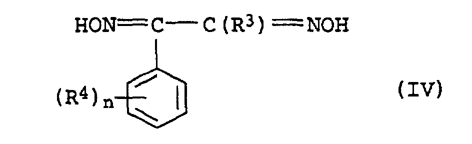 zu einer Verbindung der Formel V 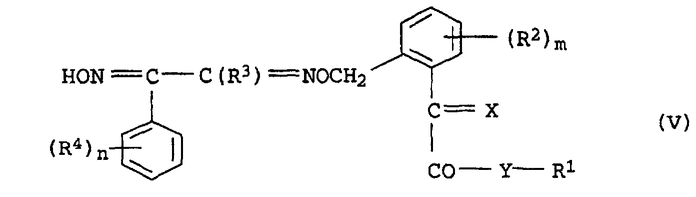 umsetzt und V anschließend mit einer Verbindung der Formel VI
R⁵-L² VI
in der L² für eine nucleophil austauschbare Abgangsgruppe steht, zu I umsetzt.

5. Verfahren zur Herstellung der Verbindungen der Formel I gemäß Anspruch 1, **dadurch gekennzeichnet, daß** man ein Benzylderivat der Formel II gemäß Anspruch 1 mit einem Carbonylhydroxyimin der Formel VII 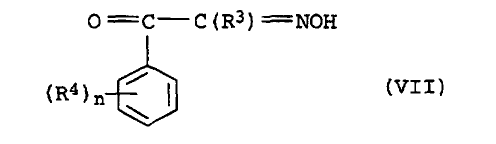 zu einer Verbindung der Formel VIII 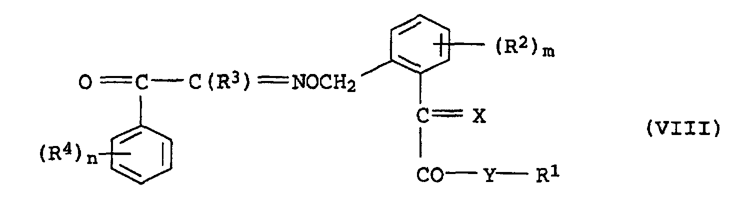 umsetzt, VIII anschließend entweder
a) zunächst mit Hydroxylamin oder dessen Salz und danach mit einer Verbindung der Formel VI (R⁵-L²) gemäß Anspruch 5 oder
b) mit einem Hydroxylamin oder einem Hydroxylammoniumsalz der Formel IXa bzw. IXb
R⁵-ONH₂ IXa
R⁵-ONH₃^{⊕} Q^{⊖} IXb
in der Q^{⊖} für das Anion einer Säure steht,
umsetzt.

6. Mittel gegen tierische Schädlinge oder Schadpilze, enthaltend übliche Zusatzstoffe und eine wirksame Menge einer Verbindung der Formel I gemäß Anspruch 1.

7. Mittel nach Anspruch 6 zur Bekämpfung tierischer Schädlinge aus der Klasse der Insekten, Spinntiere oder Nematoden.

8. Verfahren zur Bekämpfung von tierischen Schädlingen oder Schadpilzen, **dadurch gekennzeichnet, daß** man die Schädlinge oder Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

9. Verbindungen der Formel VIII gemäß Anspruch 5.

10. Verbindungen der Formel III 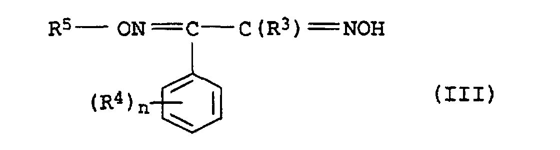 in der die Variablen die in Anspruch 2 genannten Bedeutungen haben.

11. Verbindungen der Formel IV 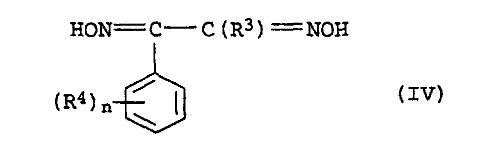 in der die Variablen die in Anspruch 2 genannten Bedeutungen haben.

12. Verbindungen der Formel VII 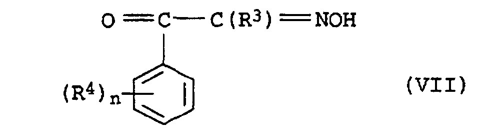 in der die Variablen die in Anspruch 2 genannten Bedeutungen haben.

13. Verbindungen der Formel VIIa 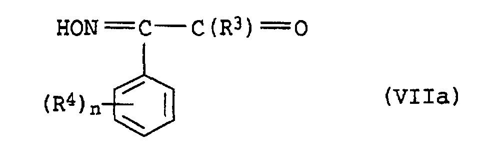 in der die Variablen die in Anspruch 2 genannten Bedeutungen haben.

14. Verbindungen der Formel X 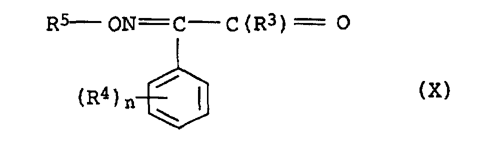 in der die Variablen die in Anspruch 2 genannten Bedeutungen haben.

15. Verbindungen der Formel XI 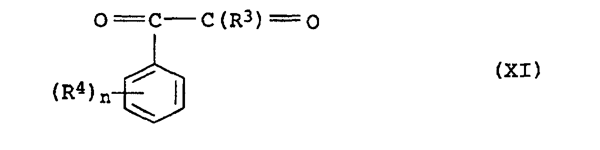 in der die Variablen die in Anspruch 2 genannten Bedeutungen haben.

## Claims

1. A phenylacetic acid derivative of the formula I 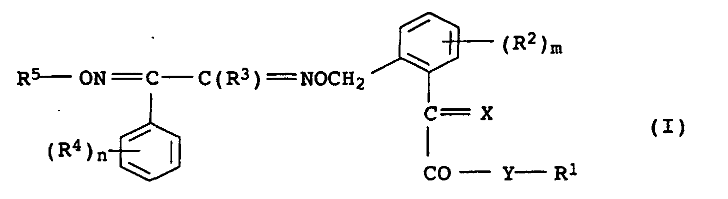 where the variables have the following meanings:
X is NOCH₃, CHOCH₃ or CHCH₃;
Y is O or NH;
R¹ is methyl;
R² is cyano, nitro, trifluoromethyl, halogen, C₁-C₄-alkyl or C₁-C₄-alkoxy;
m is 0, 1 or 2, it being possible for the radicals R² to be different if m is 2;
R³ is hydrogen, cyano, C₁-C₄-alkyl, C₁-C₄-haloalkyl, C₁-C₄-alkoxy or cyclopropyl;
R⁴ is C₁-C₄-alkylenedioxy, the alkylene groups being partially or fully halogenated, or is one of the radicals:
-NR^{c}-(C=O)-Aₚ-R^{a},
where
R^{a} is C₁-C₆-alkyl or C₂-C₆-alkenyl,
R^{c} is hydrogen or alkyl, alkenyl, alkynyl, alkylcarbonyl, alkenylcarbonyl, alkynylcarbonyl, alkoxycarbonyl, alkenyloxycarbonyl, alkynyloxycarbonyl, cycloalkyl, cycloalkylcarbonyl, cycloalkyloxycarbonyl, arylcarbonyl or hetarylcarbonyl,
p is 0 or 1 and
A is oxygen, sulfur or nitrogen, the nitrogen having attached to it hydrogen or C₁-C₆-alkyl;
n is 1 or 2, it being possible for the radicals R⁴ to be different if n is 2;
R⁵ is hydrogen,
C₁-C₆-alkylsulfonyl,
C₁-C₆-alkyl, C₃-C₆-alkenyl, C₃-C₆-alkynyl, it being possible for these radicals to be partially or fully halogenated and/or to have attached to them one to three of the following groups: cyano, nitro, hydroxyl, mercapto, amino, carboxyl, aminocarbonyl, aminothiocarbonyl, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, arylthio, hetaryl, hetaryloxy, hetarylthio, it being possible for the cyclic groups, to have attached to them one to three of the following substituents: cyano, nitro, hydroxyl, mercapto, amino, carboxyl, aminocarbonyl, aminothiocarbonyl, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C₃-C₆-alkynyloxy;
C₃-C₆-cycloalkyl, which can be partially or fully halogenated and/or, independently of each other, can have attached to it one to three of the following groups: cyano, C₁-C₆-alkyl, C₁-C₆-haloalkyl, C₂-C₆-alkenyl, C₁-C₆-alkoxy and C₁-C₆-alkylthio,
or a salt thereof.

2. A phenylacetic acid derivative of the formula I as claimed in claim 1, in which
R⁴ is C₁-C₄-alkylenedioxy, the alkylene groups being partially or completely halogenated.

3. A process for the preparation of a compound of the formula I as claimed in claim 1, which comprises reacting a benzyl derivative of the formula II 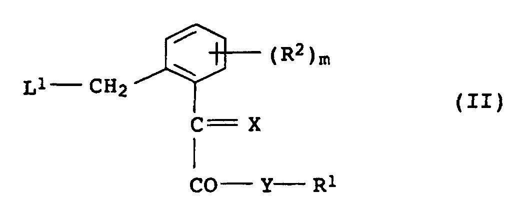 where L¹ is a nucleophilically exchangeable leaving group with a hydroxyimine of the formula III

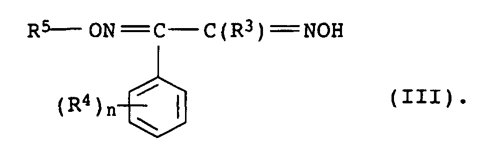

4. A process for the preparation of a compound of the formula I as claimed in claim 1, which comprises reacting a benzyl derivative of the formula II as set forth in claim 3 in a manner known per se with a dihydroxyimine of the formula IV 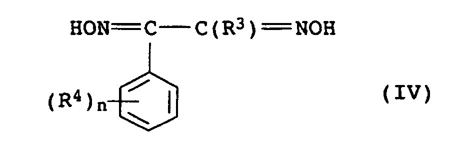 to give a compound of the formula V 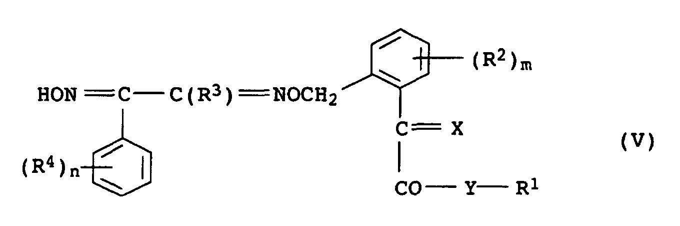 and subsequently reacting V with a compound of the formula VI
R⁵-L² VI
where L² is a nucleophilically exchangeable leaving group to give I.

5. A process for the preparation of a compound of the formula I as claimed in claim 1, which comprises reacting a benzyl derivative of the formula II as set forth in claim 3 with a carbonylhydroxyimine of the formula VII 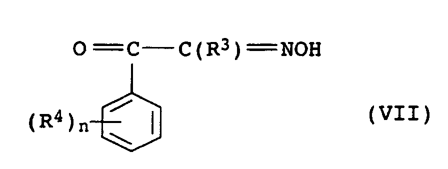 to give a compound of the formula VIII 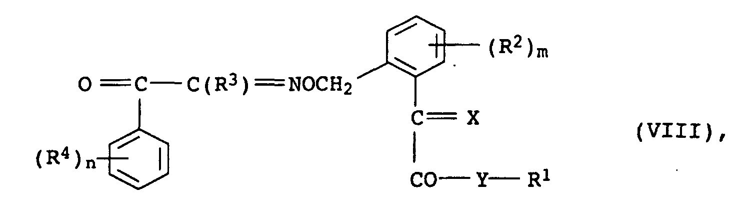 and subsequently reacting VIII
a) first with hydroxylamine or a salt thereof and then with a compound of the formula VI (R⁵-L²) as set forth in claim 4 or
b) with a hydroxylamine or a hydroxylammonium salt of the formula IXa or IXb
R⁵-ONH₂ IXa
R⁵-ONH₃^{⊕} Q^{⊖} IXb
where Q^{⊖} is the anion of an acid.

6. A composition against animal pests or harmful fungi, which comprises customary additives and an effective amount of a compound of the formula I as claimed in claim 1.

7. A composition as claimed in claim 6 for controlling animal pests from the classes of the insects, arachnids or nematodes.

8. A method of controlling animal pests or harmful fungi, which comprises treating the pests or harmful fungi, their environment, or the plants, areas, materials or spaces to be kept free from them, with an effective amount of a compound of the formula I as claimed in claim 1.

9. A compound of the formula VIII as set forth in claim 5.

10. A compound of the formula III 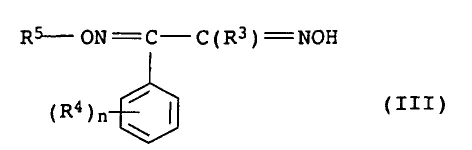 where the variables have the meanings given in claim 2.

11. A compound of the formula IV 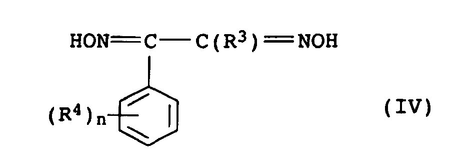 where the variables have the meanings given in claim 2.

12. A compound of the formula VII 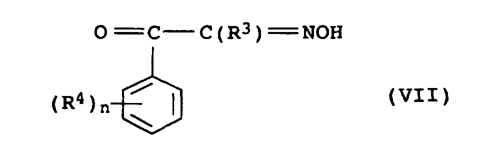 where the variables have the meanings given in claim 2.

13. A compound of the formula VIIa 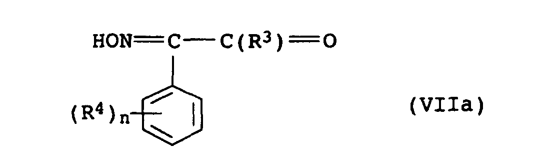 where the variables have the meanings given in claim 2.

14. A compound of the formula X 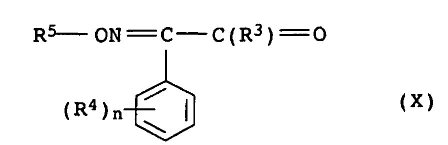 where the variables have the meanings given in claim 2.

15. A compound of the formula XI 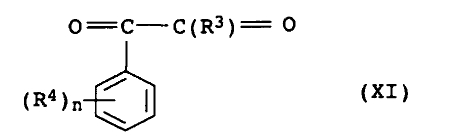 where the variables have the meanings given in claim 2.

## Revendications

1. Dérivés de l'acide phénylacétique répondant à la formule I 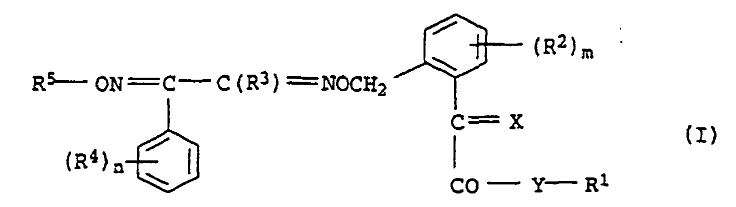 dans laquelle les symboles ont les significations suivantes :
X : NOCH₃, CHOCH₃ ou CHCH₃;
Y: O ou NH;
R¹ : un groupe méthyle ;
R² : un groupe cyano, nitro, trifluorométhyle, un halogène, un groupe alkyle en C1-C4 ou alcoxy en C1-C4 ;
m : 0, 1 ou 2, les substituants R² pouvant être différents lorsque m est égal à 2 ;
R³ : l'hydrogène, un groupe cyano, alkyle en C1-C4, halogénoalkyle en C1-C4, alcoxy en C1-C4 ou cyclopropyle ;
R⁴ : un groupe alkylènedioxy en C1-C4, la partie alkylène pouvant être halogénée en totalité ou en partie, ou l'un des groupes
-NR^{c}-(C=O)-Aₚ-R^{a},
dans lequel
R^{a} représente un groupe alkyle en C1-C6 ou alcényle en C2-C6 ;
R^{c} représente l'hydrogène ou un groupe alkyle, alcényle, alcynyle, alkylcarbonyle, alcénylcarbonyle, alcynylcarbonyle, alcoxycarbonyle, alcényloxycarbonyle, alcynyloxycarbonyle, cycloalkyle, cycloalkylcarbonyle, cycloalcoxycarbonyle, arylcarbonyle ou hétéroarylcarbonyle ;
p est égal à 0 ou 1 et
A représente l'oxygène, le soufre ou l'azote, ce dernier portant de l'hydrogène ou un groupe alkyle en C1-C6 ;
n : 1 ou 2, les substituants R⁴ pouvant être différents lorsque n est égal à 2 ;
R⁵ : l'hydrogène,
un groupe alkylsulfonyle en C1-C6,
un groupe alkyle en C1-C6, alcényle en C3-C6, alcynyle en C3-C6, ces groupes pouvant être partiellement ou totalement halogénés et/ou pouvant porter un à trois des substituants suivants : cyano, nitro, hydroxy, mercapto, amino, carboxyle, aminocarbonyle, aminothiocarbonyle, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, alcényloxy en C2-C6, cycloalkyle en C3-C6, cycloalcoxy en C3-C6, hétérocyclyle, hétérocyclyloxy, aryle, aryloxy, arylthio, hétéroaryle, hétéroaryloxy, hétéroarylthio, les groupes cycliques pouvant eux-mêmes être halogénés en totalité ou en partie et/ou pouvant porter un à trois des substituants suivants : cyano, nitro, hydroxy, mercapto, amino, carboxyle, aminocarbonyle, aminothiocarbonyle, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alcoxy en Cl-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et alcynyloxy en C3-C6 ; un groupe cycloalkyle en C3-C6 qui peut être halogéné en totalité ou en partie et/ou peut porter, indépendamment les uns des autres, un à trois des groupes suivants :
cyano, alkyle en C1-C6, halogénoalkyle en C1-C6, alcényle en C2-C6, alcoxy en C1-C6 et alkylthio en C1-C6,
et leurs sels.

2. Dérivés de l'acide phénylique de formule I selon la revendication 1 dans laquelle R⁴ représente un groupe alkylidènedioxy en C1-C4 dont la partie alkylène est halogénée en totalité ou en partie.

3. Procédé de préparation des composés de formule I de la revendication 1, **caractérisé par le fait que** l'on fait réagir un dérivé benzylique de formule II 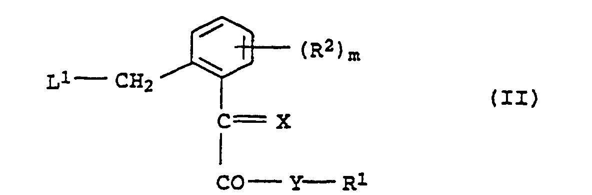 dans laquelle L¹ représente un substituant éliminable par échange nucléophile, avec une hydroxyimine de formule III

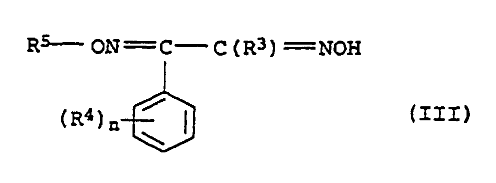

4. Procédé pour la préparation des composés de formule I de la revendication 1, **caractérisé par le fait que** l'on fait réagir de manière connue en soi un dérivé benzylique de formule II selon la revendication 1 avec une dihydroxyimine de formule IV 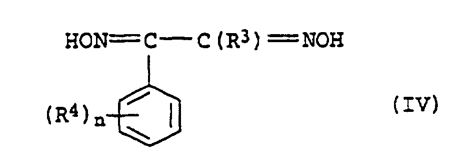 ce qui donne un composé de formule V 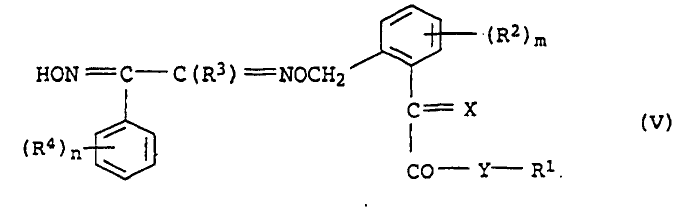 qu'on fait ensuite réagir avec un composé de formule VI
R⁵-L² VI
dans laquelle L² représente un substituant éliminable par échange nucléophile, ce qui donne un composé I.

5. Procédé de préparation des composés de formule I de la revendication 1, **caractérisé par le fait que** l'on fait réagir un dérivé benzylique de formule II de la revendication 1 avec une carbonylhydroxyimine de formule VII 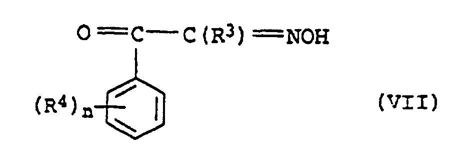 ce qui donne un composé de formule VIII 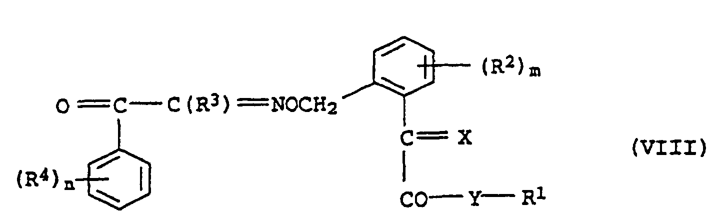 qu'on fait ensuite réagir
a) d'abord avec l'hydroxylamine ou l'un de ses sels puis avec un composé de formule VI (R⁵-L²) de la revendication 5 ou bien
b) avec une hydroxylamine ou un sel d'hydroxylammonium de formules respectives IXa et IXb
R⁵-ONH₂ IXa
R⁵-ONH₃^{⊕} Q^{⊖} IXb
dans laquelle Q^{⊖} représente l'anion d'un acide.

6. Produit pour combattre les parasites animaux ou les mycètes nuisibles, contenant des additifs usuels et une quantité efficace d'un composé de formule I de la revendication 1.

7. Produit selon la revendication 6 pour combattre les parasites animaux de la classe des insectes, des arachnides ou des nématodes.

8. Procédé pour combattre les parasites animaux ou les mycètes nuisibles, **caractérisé par le fait que** l'on traite les parasites ou les mycètes nuisibles, leur habitat ou les végétaux, aires, matériaux ou locaux qu'on veut protéger contre eux par une quantité efficace d'un composé de formule I de la revendication 1.

9. Composés de formule VIII de la revendication 5.

10. Composés de formule III 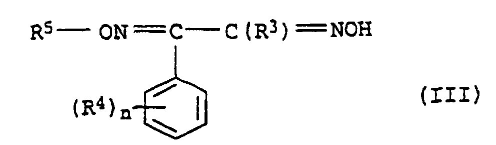 dans laquelle les symboles ont les significations indiquées dans la revendication 2.

11. Composés de formule IV 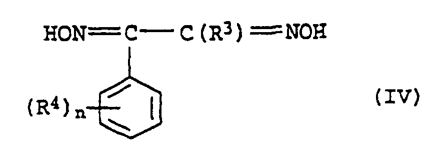 dans laquelle les symboles ont les significations indiquées dans la revendication 2.

12. Composés de formule VII 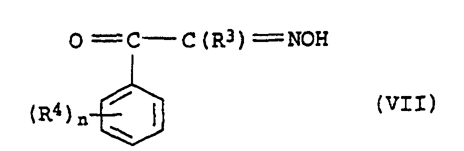 dans laquelle les symboles ont les significations indiquées dans la revendication 2.

13. Composés de formule VIIa 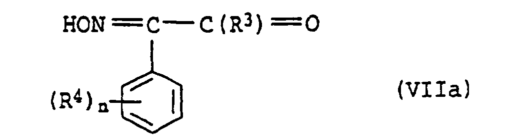 dans laquelle les symboles ont les significations indiquées dans la revendication 2.

14. Composés de formule X 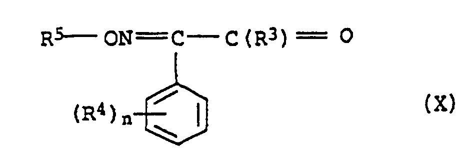 dans laquelle les symboles ont les significations indiquées dans la revendication 2.

15. Composés de formule XI 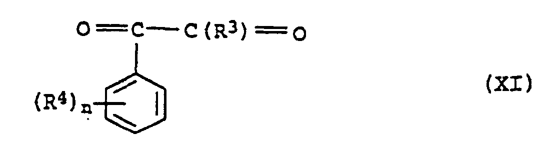 dans laquelle les symboles ont les significations indiquées dans la revendication 2.
